Europäisches Patentamt

European Patent Office

Office européen des brevets

(19)

(11) Publication number: **0 189 467**
**B1**

(12) **EUROPEAN PATENT SPECIFICATION**

(45) Date of publication of patent specification: **22.03.89**

(21) Application number: **85903762.4**

(22) Date of filing: **19.07.85**

(86) International application number:
**PCT/US85/01362**

(87) International publication number:
**WO 86/00890 13.02.86 Gazette 86/04**

(51) Int. Cl.⁴: **C 07 C 79/12, C 07 C 76/02**

(54) **NITRATION PROCESS FOR 2,3-DICHLORO-1,4-XYLENE.**

(30) Priority: **20.07.84 US 632836**

(43) Date of publication of application:
**06.08.86 Bulletin 86/32**

(45) Publication of the grant of the patent:
**22.03.89 Bulletin 89/12**

(84) Designated Contracting States:
**AT BE CH DE FR GB IT LI LU NL SE**

(56) References cited:
**EP-A-0 098 158**
**FR-A-2 310 994**
**FR-A-2 351 947**
**US-A-4 057 590**

(73) Proprietor: **RHONE-POULENC NEDERLAND B.V.**
**Draaistroom 1 Postbus 10**
**NL-1180 AA Amstelveen (NL)**

(72) Inventor: **SMITH, Oliver, Wendell**
**4608 Oak Park Road**
**Raleigh, NC 27612 (US)**

(74) Representative: **Baverstock, Michael George**
**Douglas et al**
**BOULT, WADE & TENNANT 27 Furnival Street**
**London, EC4A 1PQ (GB)**

Courier Press, Leamington Spa, England.

# EP 0 189 467 B1

**Description**

Technical Field

This invention relates to a process for the mononitration of 2,3-dichloro-1,4-xylene to produce 2,3-dichloro-5-nitro-1,4-xylene.

Background of the Invention

2,3-dichloro-5-nitro-1,4-xylene, a novel compound is a useful intermediate in the production of 3-amino-2,5-dichlorobenzoic acid, a commercially important herbicide.

As disclosed in "The Kinetics of Aromatic Nitration", R. C. Miller, D. S. Noyce, T. Vermeulen, *Industrial Engineering Chemistry*, Vol. 56, No. 6, June 1964, pp. 43—53, the most common medium for the nitration of aromatic compounds is a ternary mixed acid system of water, nitric acid and sulfuric acid.

In "Synthetic Housekeeping Nitration", G. Davis, and N. Cook, *Chemtech*, Oct. 1977, pp. 626—629, disclose a $H_2SO_4/HNO_3$ nitrating mixture in combination with a methylene chloride moderating solution for aromatic nitration. U.S. Patent 4,026,955 discloses the nitration of pentachlorobenzene with a mixture of nitric and sulfuric acids at a temperature range of 100°C—120°C. This patent discloses that at temperatures below 100°C, several production problems are encountered including inadequate production rates (Column 3, lines 50—65).

A particular disadvantage of the use of a "mixed acid" nitration mixture in the synthesis of 2,3-dichloro-5-nitro-1,4-xylene from 2,3-dichloro-1,4-xylene is in the production of excessive amounts of an undesirable dinitrated by-product i.e., 2,3-dichloro-5,6-dinitro-1,4-xylene which is difficult to separate from the desired mononitrated product.

Accordingly, it is an object of the present invention to provide a high yield process for the mononitration of 2,3-dichloro-1,4-xylene to produce 2,3-dichloro-5-nitro-1,4-xylene with a minimum of undesired by-product production. Another object of the present invention is to provide a low temperature process for the production of 2,3-dichloro-5-nitro, 1,4-xylene in high yields. These and other objects of the present invention will readily be apparent to those skilled in the art in light of the teachings herein set forth.

EP—A—98158 describes the nitration of 2,3-dichloro-1,4-xylene with 90% nitric acid at 50°C, to obtain 2,3-dichloro-5-nitro-1,4-xylene. This document does not indicate the ratio of nitric acid to dichloroxylene.

FR—A—2 310 994 describes a process for the preparation of aromatic mononitrated compounds by nitration of the aromatic compound with 70—100% by weight nitric acid. This document teaches that the weight ratio of nitric acid to the aromatic compound must not be lower than 3 for 70% nitric acid and 10 for 100% nitric acid. If this teaching is applied to the present problem, this means that for one mole of 2,3-dichloroxylene more than 14 moles of 80% nitric acid must be used.

Present invention relates to a process in the preparation of 2,3-dichloro-5-nitro-1,4-xylene which comprises adding 2,3-dichloro-1,4-xylene to an aqueous nitrating solution said solution consisting essentially of about 80% to about 98% nitric acid by weight, while maintaining the reaction mixture at a temperature ranging from 25 to 60°C, characterised in that the molar ratio of the nitric acid contained within the said nitrating solution to the amount of 2,3-dichloro-1,4-xylene added is from 3:1 to 10:1.

Detailed Description of the Invention

The starting material employed in the practice of this invention is 2,3-dichloro-1,4-xylene which is produced commercially as a by-product in the chlorination of 1,4-xylene.

The nitrating agent utilized in this invention, has a nitric acid content of from 80 to 98% by weight, preferably from 85 to 95% by weight, and most preferably 88 to 93% by weight of the total nitration mixture.

A sufficient amount of the nitrating solution is utilized to provide at least a three molar excess of nitric acid. The molar ratio of nitric acid to starting material (2,3-dichloro-1,4-xylene) must be at least about 3:1 and may be as high as about 20:1. The preferred range is from 4:1 to 10:1, and the most preferred range is from 5:1 to 7:1.

According to the process of this invention, 2,3-dichloro-1,4-xylene and the nitration acid solution are combined in the specified ratios at a rate which permits maintenance of the reaction temperature below about 60°C.

The reaction between 2,3-dichloro-1,4-xylene and the nitration acid solution is highly exothermic. To control the temperature of the reaction mixture during addition, it is necessary to add the 2,3-dichloro-1,4-xylene to the acid nitrating solution at a sufficiently slow rate to permit maintenance of the temperature of the reaction mixture within the desired range of 25°C—60°C. External or internal cooling should be provided to aid in regulating the reaction temperature. To avoid localized hot spots and for better overall control of the reaction temperature it is important that the reactants be mixed together well throughout the addition period as for example by stirring or agitation means.

In accordance with this invention, the addition is made at a rate permitting maintenance of the temperature of the reaction mixture in the range of about 25°C—60°C, preferably 40°C—60°C, and most preferably 45°—55°C. The temperature of the reaction is controlled by the rate of addition of the 2,3-dichloro-1,4-xylene. At temperatures below 25°C the reaction mixture solidifies and at temperatures above 60°C, numerous undesirable side reactions take place. At the reaction temperatures specified, the reaction

2

proceeds to approximately 100% completion. Very pure (normally in excess of 97% purity), 2,3-dichloro-5-nitro-1,4-xylene, is obtained by this procedure.

When the reaction is complete, the reaction mixture is cooled preferably by the addition of cooler water until the product solidifies. The solid product is then separated from the supernatent liquid. After separation, the water is added to the solid with heating until the solids are dissolved and the boiling point of the mixture is reached at approximately 100°C. The solution is then cooled until the pure product, 2,3-dichloro-5-nitro-1,4-xylene solidifies. Final separation of product is by filtration of centrifuging.

## Examples

A series of experiments were conducted to effect the mononitration of 2,3-dichloro-1,4-xylene using a nitric acid solution. Two concentrations of nitric acid (70 and 90%), two levels of stoichiometry (5 and 20 moles of nitric acid per mole of 2,3-dichloro-1,4-xylene) and two temperatures (25°C and 50°C) were compared in eight experiments. An additional four experiments at varying nitric acid concentrations afforded a 99% yield (GC Area %) of 2,3-dichloro-5-nitro-1,4-xylene containing 0.6% dinitrated by-product (2,3-dichloro-5,6-dinitro-p-xylene). Preferred conditions were 5 moles of nitric acid per mole of 2,3-dichloro-1,4-xylene, at 50°C and at a nitric acid concentration of 85—90%. These conditions afforded a 99% yield of 98.9% pure 2,3-dichloro-5-nitro-1,4-xylene. The following examples further illustrate the practice of this invention.

Area percent analyses of the product obtained were determined by gas chromatography. Samples were prepared by dissolving approximately 0.1 gram of sample product in methylene chloride and analyzing isothermally at 193°C. Retention times were as follows:

| Intermediates | Retention Time, Minutes |
|---|---|
| 2,3-Dichloro-p-xylene | 1.52 |
| 2,3-Dichloro-5-nitro-p-xylene | 6.97 |
| 2,3-Dichloro-5,6-dinitro-p-xylene | 25.10 |

The data is reported as a percent of the total area excluding methylene chloride.

## Examples 1—13

Runs 1 through 12 were conducted in 5 cc heavy-walled bottles heated in a heating module. Samples were quenched on wet-ice and filtered. If the product was liquid, dichloromethane was added and the sample was separated as a dichloromethane layer. The solid samples were dissolved in dichloromethane and all samples were analyzed by gas chromatography. Area percent analyses were determined for Runs 1 through 12 (TABLE I).

The final experiment, Run-13, was conducted by adding 150 grams of 2,3-dichloro-1,4-xylene (99%) to 300 grams of 90% nitric acid and not allowing the exotherm to rise above 50°C. After the 15 minute addition period, the solution was maintained for an additional 30 minutes at 50°C. The mixture was diluted with water and wet ice (200 gms), filtered, resuspended in water heated to 80°C and filtered again. The product was dried under ambient conditions for three days. The resulting yellow crystalline precipitate weighed 187.5 grams and was 98.9 percent pure by gas chromatographic analysis. The yield was 99% by gas chromatographic area percent analysis.

## Example 14

Mixed acid nitration of 2,3-dichloro-1,4-xylene

In a 1000 cc round bottom flask equipped with an addition funnel, internal thermometer and mechanical stirrer, nitric acid (90.0 gm) and sulfuric acid (300 gm) were mixed. After cooling to 10°C, 2,3-dichloro-1,4-xylene (100 gm., 0.571 m) was added slowly while maintaining the temperature between 10—12°C. Replacing the ice bath with a heating mantle led to an exotherm to 60°C. The temperature was maintained between 40 and 60°C for one hour then stirred in water. The product weighed 133.8 gm. and had a melting point of 105—109°C. Gas chromatographic analysis revealed the product was a 2:1 mixture of 2,3-dichloro-5-nitro-1,4-xylene and 2,3-dichloro-5,6-dinitro-1,4-xylene. The product yield was only approximately 66%. Moreover, a complicated separation and purification process would have to be utilized to separate the desired mononitrated product from the undesired dinitrated by-product.

3

## TABLE I

### THE NITRATION OF 2,3-DICHLORO-P-XYLENE

| VARIABLES | 1 | 2 | 3 | 4 | 5 | 6 | 7 | 8 | 9 | 10 | 11 | 12 | 13 |
|---|---|---|---|---|---|---|---|---|---|---|---|---|---|
| $HNO_3$, moles/mole of St.Mat. | 5 | 20 | 5 | 20 | 5 | 20 | 5 | 20 | 3 | 3 | 2.9 | 3 | 5 |
| Temperature, °C | 25 | 25 | 50 | 50 | 25 | 25 | 50 | 50 | 50 | 50 | 50 | 50 | 50 |
| $HNO_3$, % | 70 | 70 | 70 | 70 | 90 | 90 | 90 | 90 | 80 | 85 | 90 | 98 | 90 |

ANALYSIS (GC Area )

| | 1 | 2 | 3 | 4 | 5 | 6 | 7 | 8 | 9 | 10 | 11 | 12 | 13 |
|---|---|---|---|---|---|---|---|---|---|---|---|---|---|
| **Reaction Time - 1 hr.** | | | | | | | | | | | | | |
| Starting Material (DCX) | 99.4 | 99.4 | 98.9 | 93.5 | 0 | 0 | 0 | 0 | | | | | |
| Mononitro DCX | 0 | 0.6 | 0.9 | 6.1 | 99.5 | 97.7 | 99.5 | 99.8 | | | | | |
| Dinitro DCX | 0 | 0 | 0 | 0 | 0 | 0 | 0 | 0 | | | | | |
| **Reaction Time - 2 Hrs.** | | | | | | | | | | | | | |
| Starting Material (DCX) | 97.7 | 99.6 | 98.0 | 88.2 | 0.3 | 0 | 0 | 0 | | | | | |
| Mononitro DCX | 2.1 | 0.3 | 1.8 | 10.8 | 99.5 | 90.3 | 99.7 | 76.8 | | | | | |
| Dinitro DCX | 0 | 0 | 0 | 0 | 0 | 9.2 | 0 | 23.2 | | | | | |
| **Reaction Time - 1/2 hr.** | | | | | | | | | | | | | |
| Starting Material (DCX) | | | | | | | | | 95.7 | 71.4 | 46.3 | 4.7 | 0 |
| Mononitro DCX | | | | | | | | | 4.0 | 28.4 | 53.4 | 91.9 | 98.9 |
| Dinitro DCX | | | | | | | | | 0 | 0 | 0 | 3.3 | 0.6 |
| **Final Product Data** | | | | | | | | | | | | | |
| Starting Material (DCX) | | | | | | | | | | | | | 0 |
| Mononitro DCX | | | | | | | | | | | | | 98.9 |
| Dinitro DCX | | | | | | | | | | | | | 0.6 |
| Yield, % | | | | | | | | | | | | | 99 |

St. Mat. - Starting material (DCX) - 2,3-dichloro-1,4-xylene
Mononitro DCX - 2,3-dichloro-5-nitro-1,4-xylene
Dinitro DCX - 2,3-dichloro-5,6-dinitro-1,4-xylene

# EP 0 189 467 B1

**Claims**

1. A process for the preparation of 2,3-dichloro-5-nitro-1,4-xylene which comprises adding 2,3-dichloro-1,4-xylene to an aqueous nitrating solution said solution consisting essentially of about 80% to about 98% nitric acid by weight, while maintaining the reaction mixture at a temperature ranging from 25 to 60°C, characterised in that the molar ratio of the nitric acid contained within the said nitrating solution to the amount of 2,3-dichloro-1,4-xylene added is from 3:1 to 10:1.

2. A process according to claim 1, wherein the molar ratio of nitric acid to 2,3-dichloro-1,4-xylene is from about 4:1 to 10:1.

3. A process according to claim 1, wherein the molar ratio of nitrid acid to 2,3-dichloro-1,4-xylene is from about 5:1 to 7:1.

**Patentansprüche**

1. Verfahren zur Herstellung von 2,3-Dichlor-5-nitro-1,4-xylol, umfassend die Zugabe von 2,3-Dichlor-1,4-xylol zu einer wäßrigen Nitrierlösung, wobei die Lösung im wesentlichen besteht aus etwa 80 bis etwa 98 Gew.-% Salpetersäure, während das Reaktionsgemisch bei einer Temperatur im Bereich von 25 bis 60°C gehalten wird, dadurch gekennzeichnet, daß das Molverhältnis von Salpetersäure in der erwähnten Nitrierlösung zur zugesetzten Menge 2,3-Dichlor-1,4-xylol 3:1 bis 10:1 beträgt.

2. Verfahren nach Anspruch 1, wobei das Molverhältnis von Salpetersäure zu 2,3-Dichlor-1,4-xylol etwa 4:1 bis 10:1 beträgt.

3. Verfahren nach Anspruch 1, wobei das Molverhältnis von Salpetersäure zu 2,3-Dichlor-1,4-xylol etwa 5:1 bis 7:1 beträgt.

**Revendications**

1. Procédé de préparation du 2,3-dichloro-5-nitro-1,4-xylène, qui consiste à ajouter du 2,3-dichloro-1,4-xylène à une solution aqueuse de nitration, ladite solution comprenant essentiellement environ 80% à environ 98% en poids d'acide nitrique, tout en maintenant le mélange réactionnel à une température comprise dans l'intervalle de 25 à 60°C, caractérisé en ce que le rapport molaire de l'acide nitrique présent dans ladite solution de nitration à la quantité de 2,3-dichloro-1,4-xylène ajoutée est compris dans l'intervalle de 3:1 à 10:1.

2. Procédé suivant la revendication 1, dans lequel le rapport molaire de l'acide nitrique au 2,3-dichloro-1,4-xylène est compris dans l'intervalle d'environ 4:1 à 10:1.

3. Procédé suivant la revendication 1, dans lequel le rapport molaire de l'acide nitrique au 2,3-dichloro-1,4-xylène est compris dans l'intervalle d'environ 5:1 à 7:1.